# EUROPEAN PATENT SPECIFICATION

(11) **EP 0 993 830 B1**
(45) Date of publication and mention of the grant of the patent: **13.04.2005**
(21) Application number: 99116334.6
(22) Date of filing: 26.01.1996
(51) Int. Cl.: A61K 31/44, A61K 9/50

(54) **New stabilized galenic formulations comprising an acid labile benzimidazole compound and its preparation**
Neue stabilisierte galenische Formulierungen enthaltend ein säureempfindliches Benzimidazol und Verfahren zu ihrer Herstellung
Nouvelles compositions galéniques stabilisées comprenant un benzimidazole sensible à l'acide et son procédé de préparation

(30) Priority: 01.02.1995 ES 9500181
(43) Date of publication of application: 19.04.2000
(62) Divisional of application: 96901349.9
(73) Proprietor: LABORATORIOS DEL DR. ESTEVE, S.A., 08041 Barcelona (ES)
(72) Inventor: Ballester Rodes, Montserrat, 08023 Barcelona (ES); Van Boven, Marinus, E-08391 Tiana (ES)
(74) Representative: VOSSIUS & PARTNER

(56) References cited:
- EP-A- 0 519 144
- EP-A- 0 519 365
- EP-A- 0 567 201
- EP-B- 0 247 983
- EP-B- 0 464 006
- WO-A-92/22284
- WO-A-93/25204
- WO-A-94/02140
- WO-A-96/01624
- US-A- 4 786 505
- US-A- 5 997 903
- OKHAMAFE ET AL: 'Effect of solids-polymer interactions on the properties of some aqueous-based tablet film coating formulations.I. Moisture permeability' INTERNATIONAL JOURNAL OF PHARMACEUTICS vol. 22, 1984, pages 265 - 272, XP009014826
- 'US Pharmacopoeia USP 23', page 1477 * page 1477 *
- 'The Merck Index 1996', pages 1617 - 1547 * page 1657 * * page 1547 *
- 'Merck Chemikalien, Reagenzien, p. 1, 863', * page 1 * * page 863 *

## Description

### Field of the invention

The present invention is related to new stable pharmaceutical preparations for oral administration containing a 2 [(2-pyridyl)methylsulphinyl]-benzimidazole derivative (hereinafter referred to as "benzimidazole compound") of formula I: 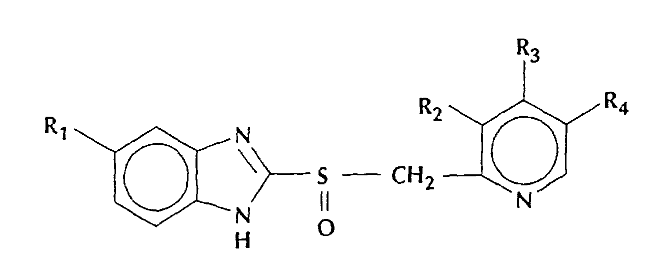 wherein R₁ is hydrogen, methoxy or difluoromethoxy, R₂ is methyl or methoxy, R₃ is methoxy, 2,2,2,-trifluoroethoxy or 3-methoxypropoxy, R₄ is hydrogen or methyl, excluding the preparation wherein the acid labile benzimidazole compound is omeprazole, when the nucleus a) is formed by coating a spherical inert core with omeprazole, hydroxy-propylmethylcellulose and talc; the inert coating b) disposed on said nucleus is formed by hydroxypropylmethylcellulose, titanium dioxide and talc; the outer layer c) disposed on the previous coating comprises an enteric coating containing co-polymerized methacryl acid methacylic acid methyl ester, triethylcitrate and talc.

The invention also relates to a method for the manufacture of such preparations and to a method for the treatment of gastrointestinal diseases.

### Background of the invention

The above benzimidazole compounds are very effective drugs for the treatment of gastric and duodenal ulcers, gastroesophageal reflux disease, severe erosive esophagitis, Zollinger-Ellison syndrome and H-pylori eradication. However, it is well known that these compounds have poor stability. In the solid state they are susceptible to heat, moisture and light, and in aqueous solution or suspension their stability decreases with decreasing pH. The degradation of these compounds is catalyzed by acidic reacting compounds.

Pharmaceutical preparations containing acid-labile compounds have to be subcoated in order to avoid a reaction between the active ingredient and the outer acidic enteric coating which reaction -if occurring- would result in degradation, destabilisation and consequently discolouration of the active ingredient.

The use of a barrier layer to protect the pharmaceutical from degradation caused by an enteric coating is well known from the prior art. Nevertheless, it is not possible to use conventional enteric coatings in a conventional way for acid labile benzimidazole compounds since decomposition takes place and the preparations become discoloured and lose in the active ingredient content with time. Prior art partially avoids the above mentioned stability problem by including an alkaline salt form of the benzimidazole compound or incorporating an alkaline reacting compound into an enteric coated preparation (magnesium oxide, hydroxide or carbonate, aluminium hydroxide, aluminium, calcium, sodium or potassium carbonate, phosphate or citrate, composite aluminium/magnesium compounds, sodium lauryl sulfate, aminoacids, N-methyl-D-glucamine, etc.) as described in US-A-4,786,505, US-A-5,232,706, EP-A-237200, EP-A-124495, US-A-5,385,739, EP-A-519144, the alkaline reacting compound being present within or on the surface of the nucleus together with the benzimidazole compound. Some authors use the alkaline reacting compound also in the composition of a second isolation layer to ensure stability of these forms. It is important to note that patent US-A-4,786,505, in its Example 1, Table 1 No.1, illustrates a formulation which is free of such alkaline compound and it is shown in Table 3 (No. 1-II) that this formulation has a rather poor stability. Thus, the association of an alkaline substance to the neutral form of the benzimidazole compound is tought in order to improve the stability of the active compound, especially for solid dosage forms, and enteric coating is recommended. That is, according to the state of the art, the addition of an alkaline substance to the pharmaceutical preparation is required to ensure the stability of the drug for long term storage.

### Outline of the invention

According to the present invention high stability solid preparations containing a benzimidazole compound of formula I are obtained. The new galenic formulations do not contain alkaline reacting compounds; thus, an alkaline reacting compound is not present in the enteric coated preparation of the invention. Surprisingly, the obtained new preparations have a significantly enhanced stability for long-term storage, much higher than the known preparations, avoiding discolouration and loss of purity, thus being more suitable for pharmaceutical use.

The new preparation is characterized in that to an inert sugar/starch spherical core, a first layer is applied containing a mixture of the benzimidazole compound of formula I as active ingredient, a water soluble inert polymer and pharmaceutical acceptable excipient with the exclusion of alkaline reacting excipients, followed by a second isolation layer formed by water soluble polymers and compatible excipients, with the exclusion of alkaline reacting excipients. Finally a third layer consisting of an enteric coating is applied. The core, the process conditions and the excipients have been selected in order to obtain the required coating efficiency for each layer.

The resulting new preparation is resistant to dissolution in acid media being stable for passage through the gastric juice, and dissolves rapidly in a neutral to alkaline media, the conditions in the proximal part of the small intestine. In fact, the acid resistance, tested as per US Pharmacopoeia, demonstrated that after 2 hours the total amount of the benzimidazole remained intact and that upon changing the pH to 6,8, after 30 minutes all the benzimidazole was dissolved (tested as per US Pharmacopoeia).

### Detailed description of the invention

In a fluidized bed apparatus, uniform spherical inert cores (composition as per US Pharmacopoeia) are coated with a first layer consisting of the acid labile benzimidazole compound, an inert water soluble polymer such as hydroxy-propylmethylcellulose or hydroxypropylcellulose, and talc. The second layer consists of an inert water soluble polymer such as hydroxypropylmethylcellulose or hydroxpropylcellulose, talc and a pigment such as titanium dioxide. The third and enteric coating layer consists of an enteric coating polymer such as copolymerized methacrylic acid / methacrylic acid methyl esters, a plasticizer such as triethylcitrate or similar plasticizers, and talc.

The layers are applied by conventional fluidized bed coating techniques using aqueous solutions or dispersions.

The active ingredients can be administered in the same dosages and according to the same protocol as the corresponding already marketed commercial dosage forms.

For oral administration, the final dosage may take the form of capsules containing the pellets, or pellets compressed into a tablet.

The dose as the benzimidazole compound lies within the range of about 1 mg to 100 mg/kg/day, adjusted to individual patients needs and for as long as clinically indicated.

The invention is described in detail in the following examples:

### Preparatory Example

In 3440 g of deionized water 436 g of omeprazole (I; R₁=-OCH₃, R₂=CH₃, R₃=-OCH₃, R₄=CH₃), 444 g of hydroxypropylmethylcellulose and 118 g of talc are dispersed.

3010 g of inert uniform sugar/starch spheres (composition according to US Pharmacopoeia) are introduced into a fluidized bed apparatus and the previous obtained dispersion is sprayed on the spheres. After spraying, the spheres are dried before applying the second layer.

In 2365 g of deionized water, 355 g of hydroxypropylmethylcellulose, 43 g of talc and 43 g of titanium dioxide are dispersed and the resulting aqueous dispersion is sprayed on the spheres obtained in the previous step. After spraying, the spheres are dried before applying the third enteric coating layer.

In 1890 g of deionized water, 1950 g of methacrylic acid copolymer (US Pharmacopoeia, type C aqueous dispersion), 98 g of triethylcitrate and 98 g of talc are dispersed, and the resulting aqueous dispersion is sprayed on the spheres obtained in the previous step. After applying this final enteric coating layer the spheres (pellets) are dried.

The pellets thus obtained were stored in closed polyethylene bags within a closed cardboard fibre container and also in closed glass containers and submitted to so called accelerated conditions, that is 40°C and 75% relative humidity. At the same time pellets obtained from Prilosec® capsules (Merck/Astra trademark) were stored in identical containers and submitted to the same conditions. The results of the test under accelerated conditions are summarized in tables 1, 2 and 3. They demonstrate a superior stability over the already authorized product on the market.

**TABLE 1**

| **COLOR OF THE PELLETS** | | | |
|---|---|---|---|
| | AT THE START | 1 MONTH | 3 MONTHS |
| Pellets (I) - Fiber container | A | A | D |
| Pellets (I) - Glass container | A | A | B |
| Prilosec - Fiber container | A | C | F |
| Prilosec - Glass container | A | A | E |
| A : White C : faint brown E : brown | | | |
| B : Pinkish white D : light brown F : Deep brown | | | |

**TABLE 2**

| **OMEPRAZOLE PURITY*** | | | |
|---|---|---|---|
| | AT THE START | 1 MONTH | 3 MONTHS |
| Pellets (I) - Fiber container | 99,5% | 98,8% | 52% |
| Pellets (I) - Glass container | 99,5% | 98,7% | 97,9% |
| Prilosec - Fiber container | 96,1% | 85,2% | 1% |
| Prilosec - Glass container | 96,1% | 96,2% | 1% |

| | | | |
|---|---|---|---|
| * Analyzed as per HPLC, described in Pharmaeuropa, Vol. 4, n° 2, June 1992 and expressed as direct area percentage | | | |

**TABLE 3**

| **OMEPRAZOLE RECOVERY AFTER US DISSOLUTION TEST** | | |
|---|---|---|
| | 1 MONTH | 3 MONTHS |
| Pellets (I) - Fiber container | 96,8% | 9,2% |
| Pellets (I) - Glass container | 99,9% | 73,8% |
| Prilosec - Fiber container | 21,3% | << 1% |
| Prilosec - Glass container | 84,5% | << 1% |

### Example according to the invention

In 580 g of deionized water, 75 g of lansoprazole (I; R₁=H, R₂=CH₃, R₃= 2,2,2-trifluoroethoxy, R₄=H), 70 g of hydroxypropylmethylcellulose and 18,5 g of talc are dispersed.

490 g of inert uniform sugar/starch spheres are introduced into a fluidized bed apparatus and the previous obtained dispersion is sprayed on the spheres. The process continues in the same manner as in Example 1 spraying the second layer and the third enteric coating layer. These two dispersions have the following composition:
Second layer: 350 g of deionized water, 52 g of hydroxypropylmethylcellulose, 7 g of talc and 7 g of titanium dioxide.
Enteric coating layer: 280 g of deionized water, 290 g of a USP methacrylic acid copolymer (type C aqueous suspension), 13 g of triethylcitrate and 13 g of talc.

The pellets obtained were stable and showed a similar profile as the ones from preparatory example.

### Biopharmaceutical studies

The purpose of the study was to investigate the bioavailability and pharmacokinetic profile of the newly developed formulation of omeprazole in comparison with the standard capsule formulation (Prilosec®; 20 mg).

Hard gelatin capsules were filled with the new galenic form of omeprazole, prepared according to example 1, in an amount corresponding to 20 mg of omeprazole.

The experimental design was a single center, open-label, randomized, 2-way cross-over study in 24 healthy male and female subjects.

Subjects reported to the clinical unit at about 8 p.m. in the evening prior to the day of treatment, and they remained hospitalized until 12 hours after drug intake. Subjects received a standard meal the evening before dosing.

The drug was given in the clinical unit with 200 ml of tap water after subjects had been fasting for at least 10 hours.

The concentration of omeprazole in blood plasma was assayed by a validated high pressure liquid chromatography method with UV detection (Internal Report No. CPR 95-742). The mean plasma concentrations are given in Table 4.

**TABLE 4**

| | | |
|---|---|---|
| The mean plasma concentrations (ng/ml) after 20 mg oral doses of omeprazole new formulation given as capsules *vs*. Prilosec® capsules. | | |

| Time (h.) | New formulation | Prilosec® |
|---|---|---|
| Baseline | 0,0 | 0,0 |
| 0,5 | 16,4 | 6,3 |
| 1,0 | 103,7 | 105,4 |
| 1,5 | 161,8 | 191,9 |
| 2,0 | 192,0 | 210,1 |
| 2,5 | 165,4 | 168,4 |
| 3,0 | 132,7 | 119,8 |
| 3,5 | 103,8 | 87,6 |
| 4,0 | 81,4 | 63,5 |
| 5,0 | 39,7 | 47,2 |
| 6,0 | 14,9 | 22,0 |
| 7,0 | 8,1 | 9,5 |
| 8,0 | 5,4 | 5,7 |
| 12,0 | 0,0 | 2,3 |

The pharmacokinetic results for omeprazole were comparable with those reported in the literature (Wilde MI, McTavish D. Omeprazole. An update of its pharmacology and therapeutic use in acid related disorders. Drugs 1994, 48: 91-132). The arithmetic mean (SD) half-lives of elimination of omeprazole were 0,9 (0,4) and 1,1 (0,7) h after oral administration of the new and Prilosec® formulation, respectively. The arithmetic mean (SD) Tₘₐₓ values of omeprazole were 2,3 (1,0) and 2,0 (1,1) h after administration of the new and Prilosec® formulation, respectively. The corresponding values for the geometric mean of the maximum plasma concentration Cₘₐₓ were 249 (197) and 241 (174) ng/ml, and those of AUC_{0-∞} were 434 (440) and 486 (436) ng h/ml, respectively.

The ratios of geometric means (new/Prilosec®) of Cₘₐₓ and AUC were 1,03 in both cases, and the 2-side 90% confidence intervals (CI) for these ratios were entirely within the interval 0,80 - 1,25. According to the CPMP guidance for bioequivalence studies, bioequivalence of the formulations (new formulation and Prilosec® formulation) can be accepted (References: CPMP Working Party on the Efficacy of Medicinal Products 1991. Note for guidance: Investigation of bioavailability and bioequivalence; Schulz HU, Steinijans VW. Striving for standards in bioequivalence assessment: A review; Int. J. Clin. Pharmacol. Ther. Toxicol. 1992, 30 (suppl.1): S1-S6).

Thus, by preparing omeprazole capsules according to the present invention, it is possible to obtain a preparation with the same bioavailability as the Prilosec® capsules containing the same amount of micronized active compound.

## Claims

1. A stable oral pharmaceutical preparation containing an acid labile benzimidazole compound of formula I: 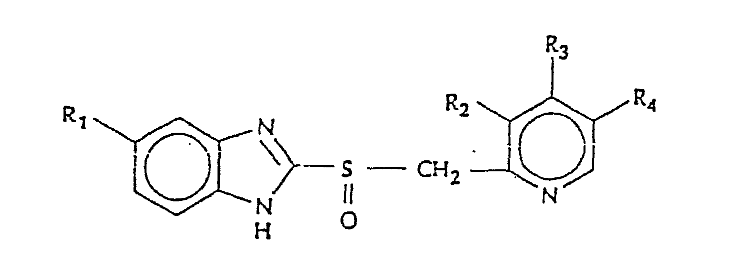 wherein R₁ is hydrogen, methoxy or difluoromethoxy, R₂ is methyl or methoxy, R₃ is methoxy, 2,2,2,-trifluoroethoxy or 3-methoxypropoxy, R₄ is hydrogen or methyl, which comprises:
(a) nucleus formed by an inert core coated with a first layer consisting of the acid labile benzimidazole, an inert water soluble polymer selected from hydroxypropylmethylcellulose and hydroxypropylcellulose and talc;
(b) an inert coating disposed on said nucleus, formed by a water soluble polymer selected from hydroxypropylmethylcellulose and hydroxypropylcellulose, talc and a pigment; and
(c) an outer layer disposed on the previous coating comprising an enteric coating consisting of a gastric resistant polymer such as co-polymerized methacrylic acid / methacrylic acid methyl esters, a plasticizer such as triethylcitrate and talc,
excluding the preparation wherein the acid labile benzimidazole compound is omeprazole, when the nucleus a) is formed by coating a spherical inert core with omeprazole, hydroxypropylmethylcellulose and talc; the inert coating b) disposed on said nucleus is formed by hydroxypropylmethylcellulose, titanium dioxide and talc; the outer layer c) disposed on the previous coating comprises an enteric coating containing co-polymerized methacryl acid methacrylic acid methyl ester, triethylcitrate and talc.

2. A process for the preparation of a stable oral pharmaceutical preparation as described in Claim 1 containing an acid labile benzimidazole compound of formula I as active ingredient, which comprises: preparing a nucleus formed by an inert core covered by a layer that contains the acid labile benzimidazole, an inert water soluble polymer comprising hydroxypropylmethylcellulose or hydroxypropylcellulose and talc; coating said nucleus with an inert layer formed by a water soluble polymer comprising hydroxypropylmethylcellulose or hydroxypropylcellulose, talc and a pigment; and finally coating the previous coating with an enteric coating consisting of a gastric resistant polymer such as copolymerized methacrylic acid / methacrylic acid methyl ester, a plasticizer such as triethylcitrate, and talc.

3. A galenic preparation in the form of capsules or tablets containing the stable oral pharmaceutical preparation according to claim 1.

## Patentansprüche

1. Stabiles orales Arzneimittel, enthaltend eine säurelabile Benzimidazol-Verbindung der Formel I: 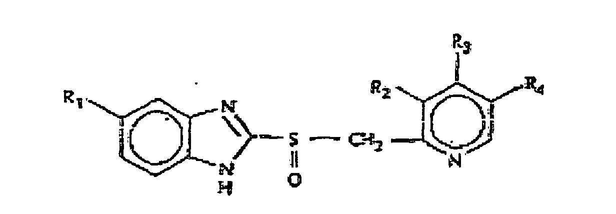 wobei R₁ Wasserstoff, Methoxy oder Difluoromethoxy ist, R₂ Methyl oder Methoxy ist, R₃ Methoxy, 2,2,2-Trifluorethoxy oder 3-Methoxypropoxy ist, R₄ Wasserstoff oder Methyl ist, umfassend:
(a) einen Kern, gebildet aus einem inerten Innenkem, überzogen mit einer ersten Schicht bestehend aus dem säurelabilen Benzimidazol, einem inerten wasserlöslichen Polymer, ausgewählt aus Hydroxypropylmethylcellulose und Hydroxypropylcellulose und Talkum;
(b) einen inerten Überzug, aufgebracht auf dem Kern, gebildet aus einem wasserlöslichen Polymer, ausgewählt aus Hydroxypropylmethylcellulose und Hydroxypropylcellulose, Talkum und einem Pigment; und
(c) einer äußeren Schicht, aufgebracht auf den vorhergehenden Überzug, umfassend einen enteralen Überzug, bestehend aus einem Magensaft-resistenten Polymer, wie copolymerisierte Methacrylsäure/Methacrylsäuremethylester, einem Weichmacher wie Triethylcitrat und Talkum,
ausgenommen das Mittel, in dem die säurelabile Benzimidazol-Verbindung Omeprazol ist, wenn der Kern a) durch Überziehen eines kugelförmigen inerten Innenkern mit Omeprazol, Hydroxypropylmethylcellulose und Talkum gebildet wird; der inerte Überzug b), aufgebracht auf dem Kern, aus Hydroxypropylmethylcellulose, Titandioxid und Talkum gebildet ist; die äußere Schicht c), aufgebracht auf den vorhergehenden Überzug, einen darmlöslichen Überzug, enthaltend copolymerisierte Methacrylsäure/Methacrylsäuremethylester, Triethylcitrat und Talkum umfaßt.

2. Verfahren zur Herstellung eines stabilen oralen Arzneimittels gemäß Anspruch 1, das als Wirkstoff eine säurelabile Benzimidazol-Verbindung der Formel I enthält, umfassend: das Herstellen eines Kerns, gebildet aus einem inerten Innenkern, überzogen mit einer Schicht, die das säurelabile Benzimidazol, ein inertes wasserlösliches Polymer, umfassend Hydroxypropylmethylcellulose oder Hydroxypropylcellulose, und Talkum enthält; Überziehen des Kerns mit einer inerten Schicht, geformt aus einem wasserlöslichen Polymer, umfassend Hydroxypropylmethylcellulose oder Hydroxypropylcellulose, Talkum und einem Pigment; und schließlich Überziehen des vorhergehenden Überzugs mit einem darmlöslichen Überzug, bestehend aus einem Magensaft-resistenten Polymer, wie copolymerisierte Methacrylsäure/Methacrylsäuremethylester, einem Weichmacher wie Triethylcitrat und Talkum.

3. Galenisches Präparat in Form von Kapseln oder Tabletten, enthaltend das stabile orale Arzneimittel gemäß Anspruch 1.

## Revendications

1. Préparation pharmaceutique orale stable contenant un composé benzimidazole acide et labile de formule I : 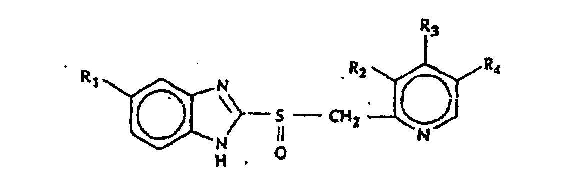 dans lequel R₁ est hydrogène, méthoxy ou difluorométhoxy, R₂ est méthyle ou méthoxy, R₃ est méthoxy, 2,2,2,-trifluoroéthoxy ou 3-méthoxypropoxy, R₄ est hydrogène ou méthyle, qui comprend :
(a) un noyau formé d'un coeur inerte enrobé par une première couche consistant en le benzimidazole acide et labile, un polymère inerte soluble dans l'eau sélectionné parmi l'hydroxypropylméthylcellulose et l'hydroxypropylcellulose, et du talc ;
(b) un enrobage inerte disposé sur ledit noyau, formé d'un polymère inerte soluble dans l'eau sélectionné parmi l'hydroxypropylméthylcellulose et l'hydroxypropylcellulose, du talc et d'un pigment ;
(c) un enrobage externe disposé sur l'enrobage précédent comprenant un enrobage entérique consistant en un polymère gastro-résistant tel que un copolymère acide méthacrylique / esters méthyliques d'acide méthacrylique, un plastifiant tel que le citrate de triéthyle et du talc ;
à l'exclusion de la préparation dans laquelle le composé benzimidazole acide et labile est l'oméprazole, quand le noyau a) est formé par enrobage d'un coeur inerte sphérique avec de l'oméprazole, de l'hydroxypropylméthylcellulose et du talc ; l'enrobage inerte b) disposé sur ledit noyau est formé par de l'hydroxypropylméthylcellulose, du dioxyde de titane et du talc ; l'enrobage externe c) disposé sur l'enrobage précédent comprend un enrobage entérique contenant un copolymère acide méthacrylique / ester méthylique d'acide méthacrylique, de citrate de triéthyle et de talc.

2. Procédé de préparation d'une préparation pharmaceutique orale stable telle que décrite dans la revendication 1 contenant un composé benzimidazole acide et labile de formule I comme ingrédient actif, qui comprend : la préparation d'un noyau formé par un coeur inerte enrobé par une couche qui contient le benzimidazole acide et labile, un polymère inerte soluble dans l'eau comprenant de l'hydroxypropylméthylcellulose ou de l'hydroxypropylcellulose et du talc ; l'enrobage dudit noyau avec une couche inerte formée par un polymère soluble dans l'eau comprenant de l'hydroxypropylméthylcellulose ou de l'hydroxypropylcellulose, du talc et un pigment ; et enfin l'enrobage de l'enrobage précédent avec un enrobage entérique consistant en un polymère gastro-résistant tel qu'un copolymère acide méthacrylique / ester méthylique d'acide méthacrylique, un plastifiant tel que le citrate de triéthyle, et du talc .

3. Préparation galénique sous forme de capsules ou de comprimés contenant la préparation pharmaceutique orale stable selon la revendication 1.
